# EUROPEAN PATENT SPECIFICATION

(11) **EP 2 197 511 B1**
(45) Date of publication and mention of the grant of the patent: **06.01.2021**
(21) Application number: 08714023.2
(22) Date of filing: 25.01.2008
(51) Int. Cl.: A61B 5/1459, G01N 33/487, G01N 21/53, A61M 1/28

(54) **APPARATUS FOR EARLY STAGE PERITONITIS DETECTION**
VORRICHTUNG FÜR DEN NACHWEIS VON PERITONITIS IM FRÜHSTADIUM
APPAREIL DE DÉTECTION PRÉCOCE DE LA PÉRITONITE

(43) Date of publication of application: 23.06.2010
(73) Proprietor: Fresenius Medical Care Holdings, Inc., Waltham, MA 02451-1457 (US)
(72) Inventor: LANDHERR, Frank, Joseph, Cary IL 60013 (US); LAN, Jay, M., Thousand Oaks CA 91320 (US)
(74) Representative: Graham Watt & Co LLP
(86) International application number: PCT/US2008/052062
(87) International publication number: WO 2009/094035

(56) References cited:
- WO-A1-2007/084849
- US-A- 3 941 121
- US-A- 5 304 173
- US-A- 6 067 157
- US-A1- 2002 037 252
- US-A1- 2005 165 276
- US-A1- 2006 184 037
- US-A1- 2007 023 334
- SHARMA.: 'Evaluation of a fiberoptic-based system for measurement of optical properties in highly attenuating turbid media.' BIOMED ENG ONLINE., [Online] vol. 5, no. 49, 23 August 2006, pages 1 - 14, XP008115581 Retrieved from the Internet: <URL:http://www.pubmedcentral.nih.gov/artic lerender.fcgitool=pubmed&pubmedid=16928274>

## Description

### Background of the Invention

The invention relates to an apparatus for medical diagnostic testing. It has application, *inter alia,* in detecting the onsite of peritonitis, for example, during continuous ambulatory peritoneal dialysis (CAPD) and automated peritoneal dialysis (APD) procedures.

Medical diagnostic testing apparatuses according to the state of the art are for instance described in WO2007/084849A1.

Peritoneal dialysis (PD) is a medical procedure for removing toxins from the blood that takes advantage of the semi-permeable membrane surrounding the walls of the abdomen or peritoneal cavity. During a PD procedure, a solution is introduced into the patient's abdomen, where it remains for up to several hours, removing blood toxins via osmotic transfer through that membrane. At completion of the procedure, the solution is drained from the body along with the toxins. CAPD is the manual form of this procedure, requiring that the patient manually drain fresh PD solution into, and spent PD solution out from, the peritoneum. In APD, the entire procedure is handled by automated equipment.

Peritonitis is a common complication of both CAPD and APD. Often caused by introduction of bacteria (e.g., from the tubing, connectors and other apparatus that make up the PD transfer set) to the peritoneum during dialysis, this swelling of the peritoneum can cause vomiting, abdominal tenderness and a host of other symptoms. Although responsive to antibiotics, peritonitis can end a patient's ability to stay on APD and CAPD therapies. In extreme cases, it can be be fatal.

Standard tests for peritonitis, usually conducted on occurrence of acute clinical symptoms, include the Gram stain procedure, performing a cell count on the peritoneal fluid, culturing that fluid, and/or performing a blood culture. Largely, these tests can only be done in the lab, after a patient has presented with symptoms. By that time, the peritonitis may well have set in, resulting in undue patient distress and potentially necessitating more extensive treatment.

More recently, reagent test strips have become available, making it possible for physicians or patient's themselves to perform more immediate diagnosis. However, test strips have a limited time window of utility and have generally not been successful in early stage detection.

CAPD and APD patients are typically counseled to maintain a keen eye for another symptom of peritonitis: a turbid or cloudy effluent bag. This can be late-developing, unfortunately, and is further compounded if the PD solution remains in the body for a long period before expulsion (as is the case, for example, during daytime dwells of APD patients). Detection of turbid effluent is further complicated in APD equipment with long drain lines, since patients may only see the effluent lines and not the effluent bag (where the turbidity is more readily apparent). Moreover, patients who are blind or have poor eyesight must rely on friends, family and/or caregivers to inspect the spent PD fluid for turbidity.

The prior art suggests that such cloudiness might be detected automatically, e.g., within APD equipment, by detecting the overall amount of non-coherent, polychromatic light that passes through a vessel of PD effluent by use of a source of such light positioned on one side of the vessel and a detector positioned at an opposing side. Implementations of this technique have generally not proven reliable because of poor signal-to-noise.

An object of the invention is to provide an improved apparatus for medical diagnosis, testing and/or treatment in the home or lab.

A still further object of the invention is to provide an improved apparatus for detecting the onset of peritonitis, e.g,. in connection with peritoneal dialysis.

Yet a still further object of the invention is to provide such apparatus as can be implemented at reasonable cost, yet, produce efficacious results.

### Summary of the Invention

The foregoing are among the objects attained by the invention which provides an automated medical testing apparatus according to claim 1 that detects the onset of peritonitis from optical characteristics of PD effluent resolved at cellular scales in the flow path Further aspects of the invention are specified in the dependent claims.

### Brief Description of the Drawings

A more complete understanding may be attained by reference to the drawings, in which:
Figures 1A - 1E depict an automated peritoneal dialysis (APD) treatment system ;
Figures 2A - 2C depict a continuous ambulatory peritoneal dialysis (CAPD) treatment system;
Figures 3A - 3B depict apparatus for testing PD effluent;
Figure 4 depicts an image of the type generated by a charge coupled device;
Figures 5A - 5C depict histograms of the type generated from images generated by charge coupled devices ;
Figures 6A - 6C show an alternate effluent flow chamber;
Figures 7A - 7B depict APD and CAPD systems utilizing apparatus for in vivo testing of peritoneal fluid; and
Figure 8 depicts further details of the in vivo testing apparatus of Figure 7.

### Detailed Description

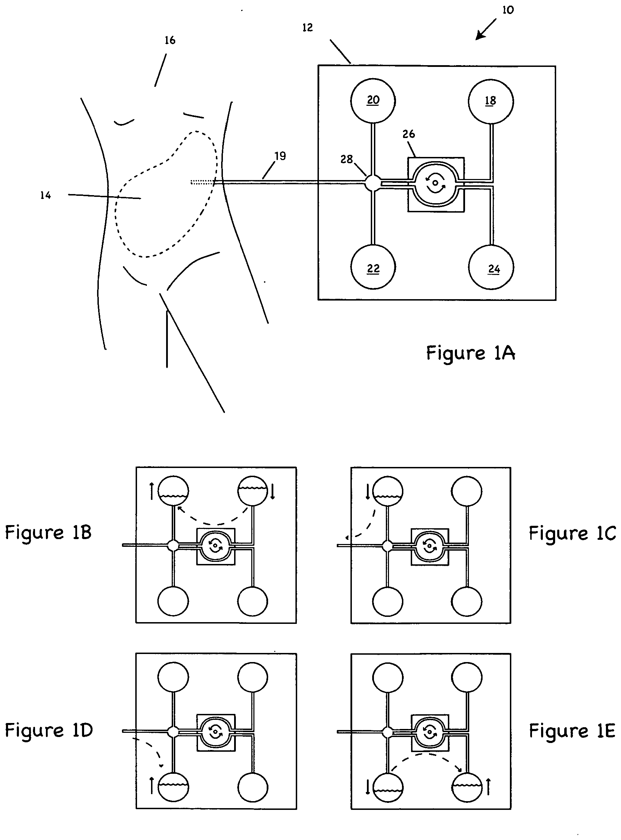

Figure 1A depicts an automated peritoneal dialysis (APD) treatment system 10 . The system 10 includes a cycler 12 or other apparatus to facilitate introducing fresh peritoneal dialysis (PD) solution into, and removing spent PD solution from, the peritoneum 14 of a patient 16.

The system 10 includes a PD solution supply chamber 18, a heating chamber 20, a weigh chamber 22, and a disposal chamber 24, all constructed an operated in the conventional manner known in the art (albeit as adapted for inclusion of PD effluent test apparatus as discussed elsewhere herein). Thus, PD supply chamber 18 holds a supply of fresh PD solution for delivery to the patient 16; heating chamber 20 brings the fresh PD solution to an appropriate temperature for delivery to the peritoneum; weigh chamber 22 hold spent PD solution expelled from the peritoneum, e.g., for weighing; and, disposal chamber 24 holds spent PD solution for disposal.

Pump 26 operates under control of a micro-controller (not shown) to move solution between the chambers 18 - 24 in the conventional manner, e.g., as illustrated in Figures 1B - 1E. Thus, for example, as shown in Figure 1B, pump 26 moves fresh PD solution from supply chamber 18 to heating chamber 20 so that the latter can bring that solution to temperature, prior to its introduction into the patient's peritoneum 14 via catheter 19. Once the desired temperature is achieved and treatment is to begin, the pump 26 opens a valve 28, allowing the heated, fresh PD solution to flow via gravity-assist into the peritoneum 14. *See,* Figure 1C.

Per Figure 1D, once the PD has dwelled for the desired period of time in the peritoneum 14, pump 26 opens valve 28 so that the spent PD solution can flow into chamber 22 for weighing (e.g., to insure that sufficient solution has be removed from the peritoneum 14), as per convention in the art. Pump 26 then moves the spent effluent from the weigh chamber 22 to the disposal chamber 24 for collection prior to disposal by the patient, health care worker, or otherwise. *See,* Figure 1D.

The conventional aspects of system 10 shown and described here are merely by way of example. It will be appreciated that apparatus for testing PD effluent (as discussed elsewhere herein) may be used in connection with APD equipment of other configurations and modes of operation than those shown in Figures 1A - 1E and described above.

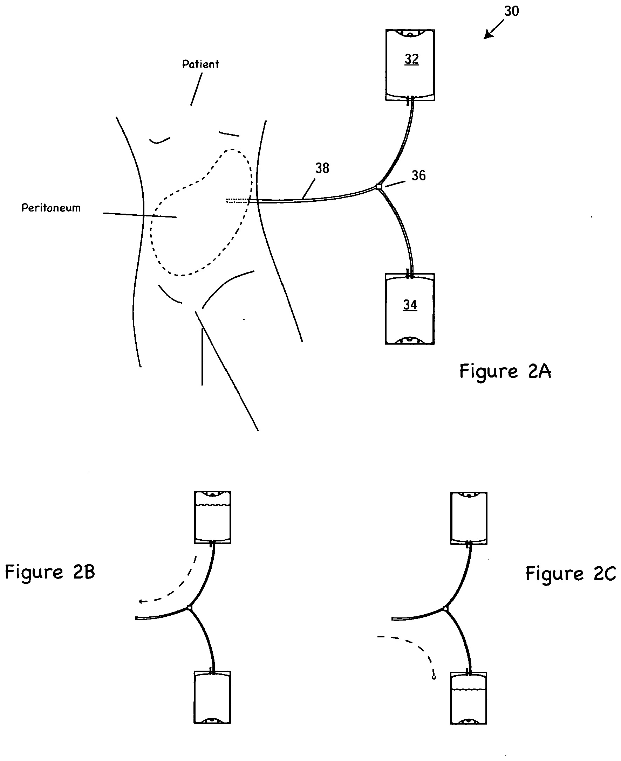

Figure 2A depicts a continuous peritoneal dialysis (CAPD) treatment system 30 . The system 30 includes a fresh PD solution supply bag 32, a spent PD solution bag 34, and a y-connector 36 for coupling those bags to peritoneal transfer set 38. The system 30 is constructed and operated in the conventional manner known in the art (albeit as adapted for inclusion of PD effluent test apparatus as discussed elsewhere herein). Thus, for example, the patient connects bags 32, 34 to the y-connector 36, as shown in Figure 2B, for a brief sterilizing flush of the connector 36. Then, as further shown in that drawing, the patient configures the connector 36 to permit fresh PD solution to flow, under gravity assist, from bag 32 into the peritoneum. Once the PD solution has dwelled for the desired period, the patient reconfigures the connector 36 to permit the spent PD solution to drain to bag 34 for disposal. *See,* Figure 2C.

The conventional aspects of system 30 shown and described here are merely by way of example. It will be appreciated that apparatus for testing PD effluent (as discussed elsewhere herein) may be used in connection with CAPD equipment of other configurations (e.g., with straight transfer tubing sets) and modes of operation than those shown in Figures 2A - 2C and described above.

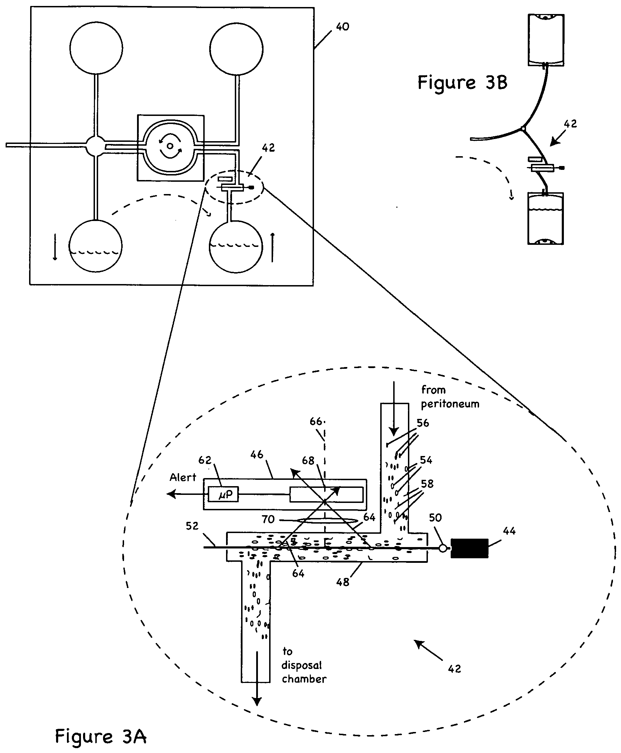

Figure 3A depicts an APD cycler 40 that is constructed and operated in the manner of cycler 12 (Figure 1), albeit including apparatus 42 for testing PD effluent (i.e., PD solution drained from the peritoneum) in a flow path of cycler 40 and/or other APD system or components of which it is a part. The cycler 40 (with test apparatus 42) can be used in place of cycler 12 in the system 10 (Figure 1), as well as in other APD treatment systems. Likewise, the test apparatus 42 can be coupled into the effluent flow path (i.e., drain lines) of the system 30 (Figure 2), as graphically depicted in inset Figure 3B, as well as in other CAPD systems. Moreover, the apparatus can be combined with kits for APD and CAPD procedures (e.g., kits that include tubing, clamps, sterilization wipes and so forth). Still further, the apparatus 42 can be coupled into fluid flow paths of laboratory, doctor's office, hospital or home test equipment and it can be sold with kits for such testing (e.g., kits that include PD effluent sample phials, drop boxes, labeling and so forth). For convenience, operation of test apparatus 42 will be described with respect to cycler 40 of Figure 3A, though, it will be appreciated that apparatus 42 can be configured and operated similarly in the aforementioned and other environments in which it is used.

By way of overview, illustrated apparatus 42 tests PD effluent in a flow path - here, the path from peritoneum 14 to disposal chamber 24 - for the onset of peritonitis and/or other conditions (e.g., the presence of blood and/or bubbles). To this end, that apparatus includes an illumination source 44 and a detector 46. The source 44 is arranged to illuminate peritoneal effluent in a chamber 48 that forms part of the flow path, and the detector 46 is arranged to detect illuminant scattered by that effluent, e.g., in a direction normal to the illuminant beam.

The source 44 and detector 46 are configured so that the detector detects reflected and/or scattered (collectively, "scattered") illuminant at a cellular scale of resolution, e.g., on a scale such that separate cellular-sized biological (or other) components in the effluent can be distinguished from one another. In applications such as early detection of the onset of peritonitis, this permits separate white blood cells (WBCs) in the effluent to be distinguished from one another (as well as from red blood cells, fibrin and other components of the effluent) so that they can be counted and so that the rate of change of those counts can be measured for purposes of detecting and signaling the onset of peritonitis. In other applications, this permits red blood cells (or other components, such as bubbles) in the effluent to be distinguished from one another (as well as from WBCs, fibrin, etc.) and counted; and so forth.

As noted, detector 46 is configured to detect illuminant scattered from the chamber 48 at a cellular scale of resolution, e.g., on a scale such that separate cellular-sized components in the effluent can be distinguished from one another. As further noted, this permits separate WBCs 54 in the effluent to be distinguished from one another (as well as from red blood cells 56, fibrin 58 and other components of the effluent) so that they can be counted and so that the rate of change of those counts can be measured for purposes of detecting and signaling the onset of peritonitis. In other applications, this permits other components - such as RBCs 56, fibrin 58, etc. - to be detected in the effluent for purposes signaling other other conditions.

The illumination source comprises a low-power laser diode generating a monochromatic collimated beam. Here, the wavelength is selected at 630 nm to coincide with an optical sensitivity of detector 46 and for suitability in reflection and scattering (collectively, as above, "scattering") from at least selected components (e.g., white blood cells) in the effluent. One may utilize lasers of other wavelengths, monochromatic or otherwise, selected in accord with foregoing or other criterion, e.g., 830 nm and 780 nm lasers, to name but a few, as well as other illumination sources, monochromatic, polychromatic, coherent and/or otherwise.

The collimated beam generated by laser diode 44 is optionally shaped by lens or columinator 50 to result in a beam 52 of gaussian or circular cross-section, though beams of other shapes may be used .

Lens 52 shapes the beam to optimize scattering from at least selected components in the effluent. This means sizing the beam at 1x - 2x and, preferably, about 1.5x the average size of the effluent components to be preferentially be detected - here, WBCs. Given an average size of 12 - 15 µm for neutrophils and eosinophils, 8 - 10 µm for lymphocytes, and 16 - 20 µm for monocytes, beam 52 of the illustrated embodiment is accordingly sized between 10 - 40 µm and, preferably, 15 - 25 µm and, still more preferably, about 20 µm. This optimizes the apparatus 42 for preferential detection of WBCs over, for example, red blood cells 56, fibrin 58 and other components of the PD effluent. One may use other beam sizes, e.g., for reason of preferential detection of other effluent components or otherwise.

The beam 52 is aimed to pass through chamber 48 in order to illuminate the effluent therein for purposes of evoking scattering from biological (and other) components in that fluid. Although the beam is aimed to pass through a center of the chamber 48, as shown, in other implementations the beam 52 may be directed otherwise.

Turning back to Figure 3A, detector 46 detects and counts scattering events - i.e., events in which illuminant is scattered from the effluent in the chamber 48 to the detector 46 - based on the intensity and/or location of those events. The detector 46 is, particularly, arranged to detect side-scattering, e.g., events within a field of view 64 centered on an axis 66 that is normal to the beam 52, as shown. In other embodiments, the detector may be arranged to detect other scattering events, e.g., back-scattering, forward-scattering, side-scattering at angles β other than normal. Thus, while in the illustrated embodiment, β is substantially 90°, more generally, β is in the range 30° - 150°; more preferably, between, 60° - 120°; still more preferably, between 80° - 100°; and, still more preferably, substantially 90°, as illustrated.

The detector 46 may employ a single-cell (or few-celled) photo diode, i.e., pin-diode 68, for purposes of detecting and signaling the occurrence of such scattering events. A lens 70 facilitates focusing the diode so that it detects those events at a cellular scale of resolution, e.g., on a scale such that separate cellular-sized biological (or other) components in the effluent can be distinguished (based on such scattering) from one another. Lens 70 is selected and arranged (vis-a-vis chamber 48 and diode 68) to preferentially focus WBCs, though, in other implementations, the lens 70 may be focused otherwise. The lens 70 is further selected and arranged for a desired depth of focus within the field of view 64, e.g., a depth of focus matching the depth of compartment 48, or a substantial portion thereof. The chamber 48 is configured to match the laser beam size and shape, e.g., so as to minimize or wholly avoid reflections (or scattering) of the beam 52 off the inner walls of the chamber itself

The laser diode 68 is selected and/or otherwise configured (e.g., through use of appropriate circuitry) to detect scattering from selected components of the effluent - here, preferentially, WBCs, though, in other applications, RBCs, fibrin, bubbles other components of the effluent. Regardless, such selection and/or configuration can be performed empirically (e.g., by testing scattering detected from an effluent of known composition) or otherwise.

Scattering events detected and signaled by the diode 60 are routed to a microprocessor 62 (or other suitable element) for analysis. This comprises counting events signaled over time and generating an alert, e.g., when the number of counts of a certain intensity (or range of intensities) varies, e.g., (i) from a baseline established for patient 16, (ii) among successive drains of spent PD solution from that patient 16, and/or (iii) when a trend of that variance over time - and, more particularly, a rate of change of counts over time (i.e., a "critical slope') - exceeds a selected amount. Such an alert can be in the form of a visible and/or audible signal to the patient 16, health-care worker, or otherwise; a hardware or other interrupt to system 12 of which test apparatus 42 forms a part; a software function call to such system; or otherwise.

One may employ a charge-coupled device (CCD), in place of pin-diode 68, for purposes of detecting and signaling the occurrence of scattering events. As above, lens 70 facilitates focusing the CCD (and obtaining a desired depth of focus) so that it detects those events at a cellular scale of resolution and, so that it preferentially focuses WBCs - though, in other embodiments, the lens 70 may be focused otherwise. In the discussion that follows, elemental designation 68 is used for the CCD, as it was for the pin-diode, since the CCD is disposed in the same functional place in apparatus 42.

As with the pin-diode, the CCD 68 is selected and/or otherwise configured to facilitate detection of scattering from selected components of the effluent (again, here, preferentially, WBCs). In this regard, the CCD 68 images the illuminated chamber 48, recording both the positions and intensities of scattering events (again, at a cellular scale of resolution) so that at least selected components (e.g., WBCs) in the effluent can be distinguished from one another and from other components of the effluent.

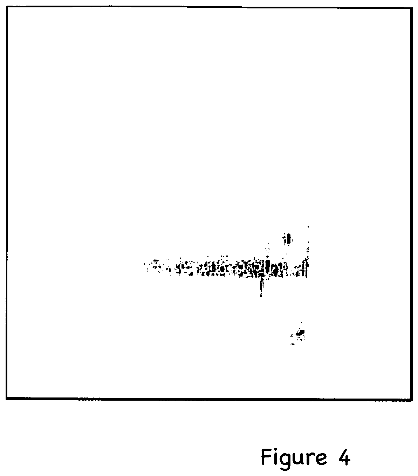

Figure 4 depicts such an image - here, generated from a simulated effluent incorporating, in lieu of WBCs, 80 glass beads (sized between 10 - 30 microns) per µL.

Images generated by the CCD are routed to the microprocessor 62 (or other suitable element) for analysis. This comprises taking a histogram of each image - or, more preferably, from multiple such images generated during drainage of spent PD solution following a single PD treatment session - with binning that is based on intensity. Depending on the number of counts in selected one(s) of the histogram bins, the microprocessor 62 can generate an alert, e.g., as discussed below.

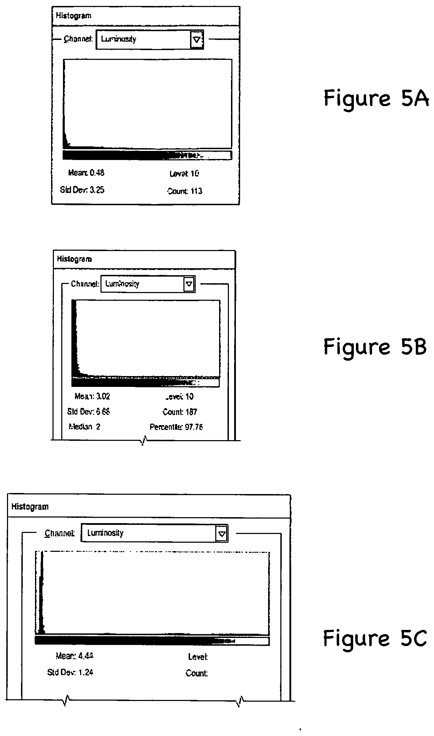

Figures 5A - 5C depict such histograms - here, generated from a simulated effluent as described above with, respectively, 40 (Figure 5A), 80 (Figure 5B) and zero (Figure 5C), glass beads per µL.

It generates that alert, e.g., when the number of counts of a certain intensity (or range of intensities) varies, e.g., (i) from a baseline established for patient 16, (ii) among successive drains of spent PD solution from that patient 16, and/or (iii) when a trend of that variance over time (i.e., from PD treatment session to session) - and, more particularly, a rate of change of counts over time (or "critical slope') - exceeds a selected amount. Again, such an alert can be in the form of a visible and/or audible signal to the patient 16, health-care worker, or otherwise; a hardware or other interrupt to system 12 of which test apparatus 42 forms a part; a software function call to such system; or otherwise.

As will be appreciated, an advantage of taking histograms from multiple CCD images is that it tends to emphasize intensity counts in the critical range. This improves the signal-to-noise ratio and, thereby, increases the efficacy of detection (e.g., of peritonitis or other conditions reflected by the effluent). Using this approach, the CCD 68 can be controlled (e.g., by the microprocessor 62 or otherwise) to acquire those multiple images during PD solution drainage by successively entering "acquisition" and "read" modes: the former, for acquiring images of the illuminated chamber 48; and the latter for reading those images to the microprocessor.

The microprocessor can perform image preprocessing prior to taking the histograms. Thus, for example, it can eliminate pixel values representing scattering from effluent components that are too long (e.g., fibrin) or too short (e.g., RBCs) - both, by way of example, to count WBCs for purposes of peritonitis detection. Further such preprocessing may be selected depending upon the specifics of the application .

### Self-Cleaning Chamber

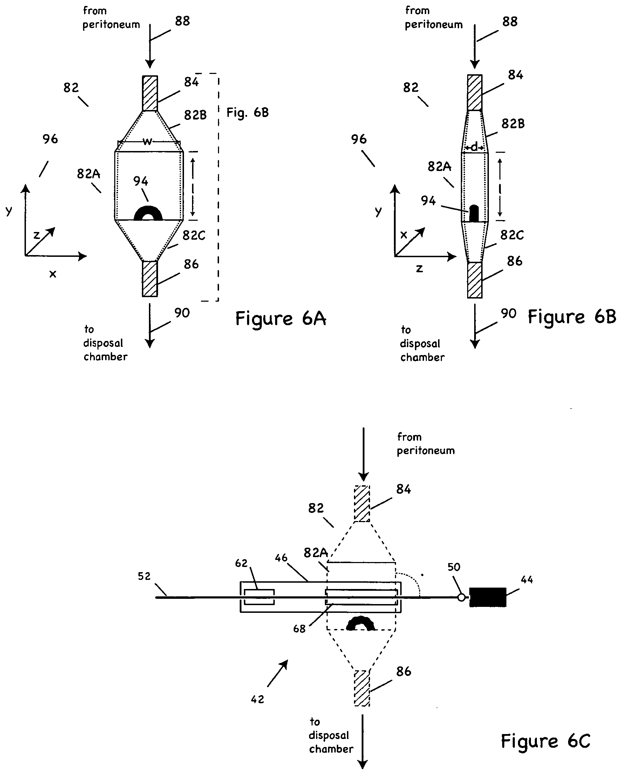

Figures 6A - 6C depict a chamber 82 for use with apparatus 42. This is an alternate to the chamber 48 shown in Figure 3A and discussed above. Thus, as with chamber 48, chamber 82 forms part of the flow path for peritoneal effluent and can be arranged so that effluent flowing through it can be illuminated by source 44 and detected by detector 46, all as described above with respect to chamber 48 in view of the discussion below.

Figures 6A and 6B depict front and side views, respectively, of chamber 82. Figure 6C illustrates a configuration of a system showing chamber 82 in combination with source 44 and detector 46.

Referring to Figures 6A and 6B, chamber 82 includes a fluid inlet port 84 and outlet port 86 for coupling into the effluent flow path of cycler 40, system 30, or other APD or CAPD systems as described above, with effluent fluid flow in the direction of arrows 88, 90 from the peritoneum (or "transfer side") to the disposal chamber ("or drain side"). The ports 84, 86 are dimensioned for attachment of tubing otherwise defining such effluent flow path in such APD or CAPD systems; though, it will be appreciated that the sizing and configuration of the ports may vary.

Chamber 82 defines a region in which effluent flowing in port 84 passes prior to exit via port 86. The chamber 82 includes a central portion 82A with inner walls (indicated by dashed lines) defining a fluid flow region generally characterized as a rectangular parallelepiped, or cuboid, with dimensions 1 (length), w (width), and d (depth).

The length 1 and width w are generally of the same size, and both are greater in size than the depth d; however, other implementations may vary in these regards. 1 and w may both be less than or equal to about 5" and, preferably, less than or equal to about 3" and, still more preferably, less than or equal to about 1", while d is less than or equal to about 1" and, more preferably, less than or equal to about 1/2" and, still more preferably, less than or equal to about 1/4". Thus, and w may be about 1" and d is about 1/4".

The central portion 82A is coupled with port 84, on the inlet side, via a proximal portion 82B having inner walls (dashed lines) with a shape generally characterized as a pyramidal frustum; and, on the outlet side, via distal portion 82C having inner walls (dashed lines) also with a shape generally characterized as a pyramidal frustum. The dimensions of the frustum defined by the inner walls of portion 82B at the proximal end (i.e., closer to the inlet) substantially match the inner diameter of the port 84 and, at the distal end (i.e., close to the outlet), substantially match the dimensions of cuboid defined by the inner diameter of portion 82A. Likewise, the dimensions of the frustum defined by the inner walls of portion 82C at the distal end (i.e., closer to the outlet) substantially match the inner diameter of the port 86 and, at the proximal end (i.e., close to the inlet), substantially match the dimensions of cuboid defined by the inner diameter of portion 82A.

Central portion 82A of the illustrated embodiment is optically clear, permitting illumination of peritoneal effluent in chamber 82 by source 44 and detection of illuminant scattered thereby by detector 46, as discussed above. To these ends, at least central portion 82A can be fabricated from molded plastics such as acrylic, polycarbonate and/or polystyrene, which have optical transmission of 450 nm - 890 nm (with maximum optical loss of around three percent) and are therefore suitable for use with a source 44 operating at a wavelength of 630 nm (+/- 20nm). Of course, other plastics or materials (such as glass, ceramics, etc.) that are optically transparent in the wavelengths discussed herein and earlier may be used as well or in addition.

The index of refraction of the plastic or other material from which central portion 82A is fabricated (within the constraints discussed above) has little significant impact on illumination of peritoneal effluent in chamber 82 by source 44 and detection of illuminant scattered thereby by detector 46. However, e.g., where a portion of region 82A serves as a lens (intentionally or otherwise), the index of refection of that plastic or other material may be more significant.

Likewise, the dispersion factor of the plastic or other material from which central portion 82A is fabricated (again, within the constraints discussed above) has little significant impact on illumination of peritoneal effluent in chamber 82 by source 44 and detection of illuminant scattered thereby by detector 46. This is particularly true where (i) the beam 52 transits the walls of central portion 82 at an angle Ω that is substantially normal to the surfaces thereof, (ii) the beam is collimated, and (iii) the beam has a diameter (and is positioned) so that no portion of it comes in contact with the sides of the central portion 82A - other than the points of transit. However, where the beam transits the walls of the central at an angle Ω other than substantially 90o central portion 82A can be fabricated from materials with lesser dispersion factors.

Although portions 82B, 82C of compartment 82 can be fabricated from the same plastic or other material as portion 82A, proximal and distal portions 82B, 82C need not be optically clear and, hence, can be fabricated from other materials, as well.

A deflector 94 is disposed at the distal end of the interior chamber 82, as shown. In the illustrated embodiment, it comprises an arc- or hemispherically-shaped member positioned in a central region of the effluent flow path downstream of a region illuminated by beam 52. Thus, for example, the deflector may be disposed at the distal end of chamber 82, yet, substantially centered vis-a-vis the x- and z-axes 96 (or, put another way, vis-a-vis the width w and depth d dimensions).

The deflector 94, which may be coupled or integral to the distal portion 82, has a rounded portion (shown as a thick, dark, curved region in the drawing) that protrudes into the distal end of the central portion 82A, as shown. Deflectors of other shapes suitable for breaking up the effluent flow (e.g., from lamellar to turbulent) in the manner discussed below may be used, as well or in addition to deflector 94 shown here. Deflector 84 may be fabricated from the same plastic or other materials as portions 82A, 82B and/or 82C, though it may be fabricated from other materials as well. Deflector 84 may bear a coating of teflon or other substance that resists adherence of biological and other materials in the effluent flow.

As a consequence of the configuration of its inner walls, chamber 82 induces lamellar flow in effluent at the proximal end of the central portion 82A. The deflector 94 provides a transition in that flow at the distal end of the central portion 82A, as well as in outlet (or distal) portion 82C, from lamellar to turbulent by breaking up the boundary layer, which causes the Reynold's Number to increase. When the Reynold's number reaches 100, eddies start to form in the effluent. Those eddies increase as the Reynold's number gets larger. The eddies help clean the inner walls of the chamber 82 - particularly, for example, at the distal end of the central portion 82A, as well as in distal portion 82C - preventing white blood cells, red blood cells, and other components of the effluent from adhering to those inner walls and/or removing those that have already adhered. Thus, the deflector may be arranged to effect a flow of effluent having a Reynold's Number that is about 100 or higher and, more preferably, about 250 or higher.

### In Vivo Testing

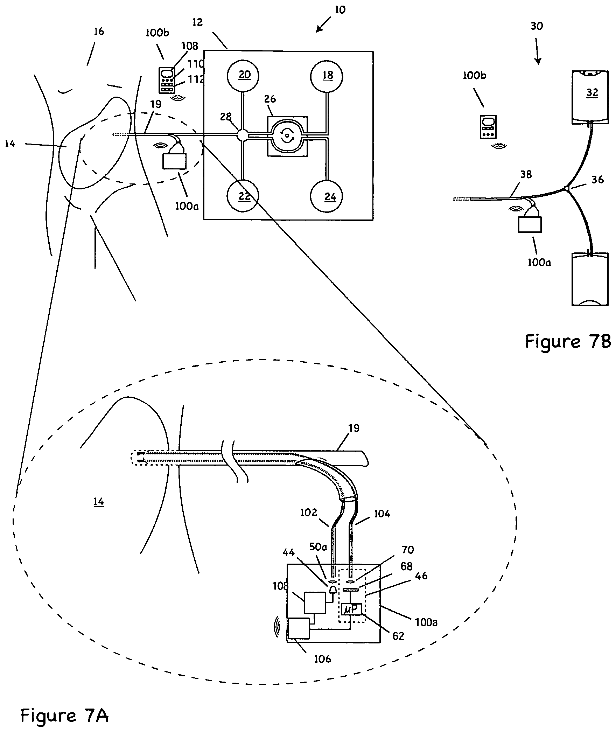

Figure 7A depicts an APD cycler 40 that is constructed and operated in the manner of cycler 12 (Figure 1), albeit including apparatus 100a for detection of peritonitis by testing peritoneal fluid in vivo, i.e., in the patient's peritoneum. The cycler 40 (with test apparatus 100a) can be used in place of cycler 12 in the system 10 (Figure 1), as well as in other APD treatment systems. Likewise, the test apparatus 100a can be used with system 30 (Figure 2), as graphically depicted in inset Figure 7B, as well as in other CAPD systems. Moreover, the apparatus 100a can be utilized as a stand-alone test apparatus (e.g., for testing fluids in a patient's body) and/or it can be combined with kits for APD, CAPD or other medical procedures. Still further, the apparatus 100a can be used, alone or in combination with the foregoing, in laboratory, doctor's office, hospital or home test equipment. For convenience, operation of test apparatus 100a will be described with respect to cycler 40 of Figure 3A, though, it will be appreciated that apparatus 100a can be configured and operated similarly in the aforementioned and other environments in which it is used.

Illustrated apparatus 100a generally operates in the manner of test apparatus 42, discussed above, albeit testing peritoneal fluid in vivo for the onset of peritonitis and/or other conditions (e.g., the presence of blood and/or bubbles) - in the abdomen and, more particularly, in the peritoneum 14 of the patient 16 - rather than in chamber 48. In addition, apparatus 100a provides for measurement of characteristics of the peritoneal fluid, e.g., its index of refraction.

When used with APD and CAPD systems (e.g., as shown in Figures 7A - 7B), this testing is typically conducted during PD treatment - i.e., at a time when the peritoneum is filled with a mix of bodily fluids (e.g., creatin and urea) and PD solution. The apparatus 100a may also be used pre- or post-treatment to test peritoneal fluid that comprises substantially only bodily fluids. In light of the latter, it will be appreciated that the term "peritoneal fluid" as used herein in regard to the content of the peritoneum refers to any combination of fluids (e.g., bodily fluids, PD solution, or a mix thereof), unless otherwise evident from context.

To these ends, apparatus 100a utilizes a first fiber optic bundle 102 to carry illuminant down catheter 19 from illumination source 44 (external to the patient) into the peritoneum 14. A second fiber optic bundle 104 carries illuminant scattered by peritoneal fluid in the peritoneum, e.g., in a direction normal to the illuminant beam, back up the catheter 19 to the detector 46 (also external to the patient). Where apparatus 100a is used with APD and CAPD systems, the bundles may be routed through transfer sets 38, as well.

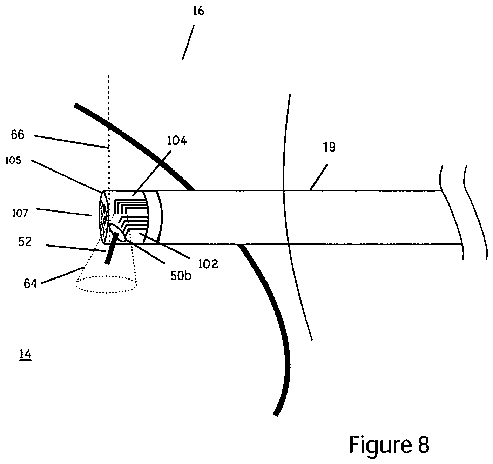

Referring to Figure 8, the distal ends of bundle 102 and bundle 104 are secured in catheter tip 105 so that the latter captures and transmits to detector 46 reflected and/or scattered (collectively, "scattered") illuminant - preferably, at a cellular scale of resolution, e.g., on a scale such that separate cellular-sized biological (or other) components in the peritoneal fluid can be distinguished from one another. As noted above, in applications such as early detection of the onset of peritonitis, this permits separate white blood cells (WBCs) in the peritoneal fluid to be distinguished from one another (as well as from red blood cells, fibrin and other components of the fluid) so that they can be counted and so that the rate of change of those counts can be measured for purposes of detecting and signaling the onset of peritonitis. In other applications, this permits red blood cells (or other components, such as bubbles) in the fluid to be distinguished from one another (as well as from WBCs, fibrin, etc.) and counted, and so forth.

Tip 105 may be constructed of plastic, ceramic, metal or other materials (or combination thereof) suitable for use in medical application for securing the bundles 102, 104. In the illustrated embodiment, it comprises a hood-shaped structure with a hollow central region in which the distal ends of bundles 102, 104 are disposed (as shown) and through which peritoneal fluids, as well as fresh and spent dialysate, may freely pass. An open "face" region of the hood-shaped structure and apertures 107 at its distal end (or "top") further facilitates such passage of fluids. In addition, the open face and apertures help insure that distal tips 102, 104 are exposed to a representative mix of fluids from the peritoneum. Those skilled in the art will of course appreciate that other structures and/or arrangements may be used in addition to, or in place of, tip 105.

Fiber optic bundles 102, 104 can be routed from apparatus 102 to peritoneum 14 through catheter 19 in the manner shown, e.g., entering the catheter 19 via an integral catheter branch 19a. Alternatively, the bundles 102, 104 can enter the catheter via a y-connector, via direct insertion into the wall of the catheter 19, or via other techniques known in the art for the routing of fiber optic bundles or other such structures through catheters into a patient's body. The bundles 102, 104 are comprised of conventional fiber optic fibers suitable for use in medical applications. Each bundle, which is sized to that it (and its partner) may pass through the catheter 19 without unduly impeding the flow of dialysate therethrough, comprise between one and hundreds, or more, of fibers, depending on individual fiber size.

As above, illumination source 44 of apparatus 100a comprises a low-power laser diode generating a monochromatic collimated beam. The wavelength is selected at 630 nm to coincide with an optical sensitivity of detector 46 and for suitability in reflection and scattering (collectively, as above, "scattering") from at least selected components (e.g., white blood cells) in the peritoneal fluid. As noted earlier, one may utilize lasers of other wavelengths, monochromatic or otherwise, selected in accord with foregoing or other criterion, e.g., 830 nm and 780 nm lasers, to name but a few, as well as other illumination sources, monochromatic, polychromatic, coherent and/or otherwise. Source 44 can be powered and otherwise driven by illustrated drive circuitry 108 in the conventional manner of laser diode drive circuitry known in the art - as adapted in accord with the teachings hereof.

As above, the collimated beam generated by laser diode 44 of apparatus 100a can be shaped by lens or columinator 50a for transfer down bundle 102 to the peritoneum 14. A further lens or columinator 50b can be provided at the distal end of the bundle 102 to shape the laser beam 52 in peritoneal cavity 14 in a gaussian or circular cross-section, though beams of other shapes may be used .

As above, lens 50b shapes the beam 52 to optimize scattering from at least selected components in the peritoneal fluid. This means sizing the beam at 1x - 2x and, preferably, about 1.5x the average size of the fluid components to be preferentially be detected - here, WBCs. Given an average size of 12 - 15 µm for neutrophils and eosinophils, 8 - 10 µm for lymphocytes, and 16 - 20 µm for monocytes, beam 52 accordingly sized between 10 - 40 µm and, preferably, 15 - 25 µm and, still more preferably, about 20 µm. This optimizes the apparatus 42 for preferential detection of WBCs over, for example, red blood cells 56, fibrin 58 and other components of the peritoneal fluid. One may use other beam sizes, e.g., for reason of preferential detection of other peritoneal fluid components or otherwise.

As above, the beam 52 emanating from the bundle 102 is aimed to pass through the peritoneum 14 in order to illuminate peritoneal fluid therein for purposes of evoking scattering from biological (and other) components in that fluid.

As above, illuminant scattered by peritoneal fluid in the peritoneum 14, e.g., in a direction normal to beam 52 is transmitted along the catheter 19 to the detector 46 by fiber optic bundle 104. From that illuminant, detector 46 determines the index of refraction of the peritoneal fluid, in addition to detecting and counting of scattering events (i.e., events in which illuminant is scattered by fluid in the peritoneum 14 to the distal end of bundle 104), based on the intensity and/or location of those events.

The distal end of bundle 104 is arranged to capture (and transmit to the detector 46 for detection) side-scattering, e.g., events within a field of view 64 centered on an axis 66 that is normal to the beam 52, as shown. In other implementations, the distal end of the bundle is arranged to capture (and transmit to the detector for detection) other scattering events, e.g., back-scattering, forward-scattering, side-scattering at angles β other than normal. Thus, while in the illustrated embodiment, β is substantially 90o, more generally, β is in the range 30o - 150o; more preferably, between, 60o - 120o; still more preferably, between 80o - 100o; and, still more preferably, substantially 90o, as illustrated.

The detector 46 employs a single-cell (or few-celled) photo diode, i.e., pin-diode 68, for purposes of detecting and signaling the occurrence of such scattering events. A lens 70 facilitates focusing the diode so that it detects those events at a cellular scale of resolution, e.g., on a scale such that separate cellular-sized biological (or other) components in the peritoneal fluid can be distinguished (based on such scattering) from one another. Lens 70 is selected and arranged (vis-a-vis the proximal end of bundle 104 and diode 68) to preferentially focus WBCs, though the lens 70 may be focused otherwise. The lens 70 is further selected and arranged for a desired depth of focus vis-a-vis field of view 64 , e.g., a depth of focus from about a few centimeters to about a few inches .

As above, the laser diode 68 is selected and/or otherwise configured (e.g., through use of appropriate circuitry) to detect scattering from selected components of the peritoneal fluid - here, preferentially, WBCs, though, in other embodiments, RBCs, fibrin, bubbles other components of the fluid. Regardless, such selection and/or configuration can be performed empirically (e.g., by testing scattering detected from a fluid of known composition) or otherwise.

As above, one may employ a charge-coupled device (CCD), in place of pin-diode 68, for purposes of detecting and signaling the occurrence of scattering events. And, too, lens 70 facilitates focusing the CCD (and obtaining a desired depth of focus) so that it detects those events at a cellular scale of resolution and so that it preferentially focuses WBCs - though the lens 70 may be focused otherwise. In the discussion that follows, elemental designation 68 is used for the CCD, as it was for the pin-diode, since the CCD is disposed in the same functional place in apparatus 100a.

As with the pin-diode, the CCD 68 is selected and/or otherwise configured to facilitate detection of scattering from selected components of the peritoneal fluid (again, here, preferentially, WBCs) and for determination of the index of refraction of the peritoneal fluid. In this regard, the CCD 68 images the illuminated peritoneal fluid (via fiber optic bundle 104), recording both the positions and intensities of scattering events (again, at a cellular scale of resolution) so that at least selected components (e.g., WBCs) in the peritoneal fluid can be distinguished from one another and from other components of that fluid.

As above, scattering events detected and signaled by the diode 60 are routed to microprocessor 62 (or other suitable element) for analysis. This comprises counting events signaled over time and generating an alert, e.g., when the number of counts of a certain intensity (or range of intensities) varies, e.g., (i) from a baseline established for patient 16, (ii) among successive drains of spent PD solution from that patient 16, and/or (iii) when a trend of that variance over time - and, more particularly, a rate of change of counts over time (i.e., a "critical slope') - exceeds a selected amount. Such an alert can be in the form of a visible and/or audible signal to the patient 16, health-care worker, or otherwise; a hardware or other interrupt to system 12 of which test apparatus 100a forms a part; a software function call to such system; or otherwise. In addition, the microprocessor determines the index of refraction of the fluid in the peritoneum 14 based on the intensity of the scattering events detected by diode 60.

In Figures 7A - 7B, such alerts and index of refraction measurements are transmitted from apparatus 100b to a remote interface unit 100b. Such transmission can be over a wireless or wired connection, or a combination thereof. Wireless transmission is employed via module 106, which utilizes the Bluetooth® wireless protocol to communicate (i.e., transmit and receive information) with remote unit 100b. One may use infrared, 802.11, or other wireless communication technologies (including long-distance wireless technologies, like satellite) instead or in addition. Still others may utilize wired connections (such as USB cables, Ethernet, and so forth), in combination with or exclusive of such wireless technologies.

Apparatus 100b, which can be sized and configured to be slipped onto/into a pocket or "worn" by the patient 16, a health care provider, or otherwise, receives such alerts index of refraction measurements for display on LCD or other display screen 108 and/or LEDs or other indicators 110. To this end, the apparatus 100b can employ a microprocessor or other circuitry (not shown) for further analysis of the alerts and information from apparatus 100a regarding scattering events, index of refraction measurements and other information (e.g., regarding battery levels and/or other aspects of the operational status of apparatus 100a). Indeed, such a microprocessor or other circuitry resident in apparatus 100b can provide some or all of the functionality of microprocessor 62, thereby, reducing the circuitry requirements of apparatus 100a.

More generally, the microprocessor or other circuitry of resident in apparatus 100b can control apparatus 100a, e.g., activating detector 46 and/or drive circuitry 108. To this end, apparatus 100b provides a keyboard 112 for accepting input from the patient, health care provider or others for activating the unit, entering codes for pairing Bluetooth module 106 or otherwise coupling/decoupling apparatus 100a and 100b for communications, specifying operational modes for detector 46 and/or drive circuitry 108 (and, more generally, apparatus 100a and 100b), and/or otherwise.

The circuitry of apparatus 100a and 100b can be powered by batteries, such as by way of non-limiting example two flat "watch type" batteries (not shown), or otherwise. Furthermore, that circuity can be implemented using surface mounted printed circuit board technology, or otherwise, in order to conserve space. As such, apparatus 100a is approximately 1.5" x 1" x 0.5", though, the dimensions may vary. Likewise, apparatus 100b is approximately 3" x 2" x 1", though, again, the dimensions may vary.

The invention is defined in the appended claims.

## Claims

1. Apparatus (42) for in vivo testing of peritoneal fluids in a patient's body, comprising
A. an illuminant source (44) and illuminant detector (46), both disposed external to a patient,
B. a first fiber optic bundle (102) configured to carry illuminant from the illuminant source into the patient's peritoneum (14) and arranged to illuminate peritoneal fluid therein,
C. a second fiber optic bundle (104) (i) having a distal end configured to capture illuminant reflected and/or scattered (collectively, "scattered") by peritoneal fluid in the patient's peritoneum and (ii) being configured to carry that captured, scattered illuminant to the detector,
D. the detector is configured to detect scattering events in the captured, scattered illuminant carried by the second fiber optic bundle at a cellular scale of resolution such that white blood cells (WBCs) in the peritoneal fluid can be (i) distinguished from one another as well as from red blood cells, fibrin and other components of the peritoneal fluid, and (ii) counted, and wherein
E. the detector includes a microprocessor (62) arranged to analyze the detected scattering events over time and to generate an alert indicative of an onset of peritonitis when any of (i) a number of counts of those events varies from a baseline and (ii) a trend of that variance over time exceeds a selected amount.

2. The apparatus of claim 1, wherein any of the first and second fiber optic bundles (102; 104) are configured to be routed to the patient's body via a catheter (19), and, optionally, any of
i) the first and second fiber optic bundles are configured to be disposed at least partially in the catheter, and, further optionally,
ii) the apparatus includes a tip (105) configured to be disposed at a distal end of the catheter for securing distal ends of the first and second fiber optic bundles such that the latter captures and transmits to the detector the illuminant scattered in the patient's body.

3. The apparatus of claim 1, comprising any of a lens and a columinator (50) disposed at a proximal end of the first fiber optic bundle configured to shape illuminant generated by the source for transfer to the first fiber optic bundle.

4. The apparatus of claim 1, comprising a columinator (50) disposed at a distal end of the first fiber optic bundle configured to shape illuminant transmitted thereby into the patient's body.

5. The apparatus of claim 1, wherein the distal end of the second optic fiber bundle is arranged to capture illuminant side-scattered by biological and other components in the patient's body.

6. The apparatus of claim 5, wherein the distal end of the second optic fiber bundle is arranged to capture illuminant any of back-scattered, forward-scattered, and/or side-scattered by biological and other components in the patient's body.

7. The apparatus of claim 1, wherein the source is a laser diode (68) or other source of coherent illuminant.

8. The apparatus of claim 7, comprising an interface unit that is disposed remotely from the detector and that is configured to display information received therefrom, and further wherein, optionally, any of:
a) the remote interface (100b) is coupled with the detector by way of a wireless link, in which case, further, optionally, the wireless link is any of a Bluetooth and infrared link,
b) the remote interface is coupled with the detector by way of any of a wired link, a wireless link or a combination thereof,
c) the interface unit is configured to control any of the detector and the source,
d) the remote interface is any of sized and configured to be slipped onto/into a pocket and/or worn by the patient, a health care provider, or other person.

9. The apparatus of claim 1, arranged for in vivo testing of peritoneal fluid, wherein any of:-
a) the detector (46) is arranged to determine a refraction index of fluid in the peritoneum as a function of illuminant scattered by fluid therein,
b) the detector (46) is arranged to detect illuminant scattered from cellular-sized components of different types in the peritoneum such that those components can be distinguished from one another.

## Patentansprüche

1. Vorrichtung (42) zum in-vivo-Testen von Peritonealflüssigkeiten im Körper eines Patienten, umfassend
A. eine Beleuchtungsquelle (44) und einen Beleuchtungsdetektor (46), die beide außerhalb eines Patienten angeordnet sind,
B. ein erstes Glasfaserbündel (102), das so konfiguriert ist, dass es Licht von der Lichtquelle in das Peritoneum (14) des Patienten transportiert und so angeordnet ist, dass es die Peritonealflüssigkeit darin beleuchtet,
C. ein zweites Glasfaserbündel (104) (i), das ein distales Ende aufweist, das so konfiguriert ist, dass es ein Licht einfängt, das von der Peritonealflüssigkeit im Peritoneum des Patienten reflektiert und/oder gestreut wird (zusammen "gestreut"), und (ii) so konfiguriert ist, dass es das eingefangene, gestreute Licht zum Detektor transportiert,
D. wobei der Detektor so konfiguriert ist, dass er Streuereignisse mit einer Auflösung auf zellulärer Ebene in dem eingefangenen, gestreuten Licht detektiert, das von dem zweiten Glasfaserbündel transportiert wird, , so dass weiße Blutkörperchen (WBK) in der Peritonealflüssigkeit (i) sowohl voneinander als auch von roten Blutkörperchen, Fibrin und anderen Bestandteilen der Peritonealflüssigkeit unterschieden und (ii) gezählt werden können und wobei
E. der Detektor einen Mikroprozessor (62) enthält, der so angeordnet ist, dass er die detektierten Streuereignisse über die Zeit analysiert und einen Alarm erzeugt, der auf eine beginnende Peritonitis hinweist, wenn von einer (i) Anzahl von Zählungen dieser Ereignisse von einer Grundlinie abgewichen wird und (ii) ein Trend dieser Varianz über die Zeit eine ausgewählte Menge überschreitet.

2. Vorrichtung nach Anspruch 1, wobei jedes der ersten und zweiten Glasfaserbündel (102; 104) so konfiguriert ist, dass es über einen Katheter (19) zum Körper des Patienten geführt wird, und optional jedes
i) der ersten und zweiten Glasfaserbündel so konfiguriert sind, dass es zumindest teilweise im Katheter angeordnet ist und weiterhin optional
ii) die Vorrichtung eine Spitze (105) enthält, die so konfiguriert ist, dass sie an einem distalen Ende des Katheters angeordnet wird, um die distalen Enden des ersten und zweiten Glasfaserbündels so zu befestigen, dass das Letztere das im Körper des Patienten gestreute Leuchtmittel einfängt und an den Detektor überträgt.

3. Vorrichtung nach Anspruch 1, umfassend eine Linse und einen Koluminator (50), die an einem proximalen Ende des ersten Glasfaserbündels angeordnet sind, das so konfiguriert ist, dass es das von der Quelle erzeugte Licht zur Übertragung auf das erste Glasfaserbündel formt.

4. Vorrichtung nach Anspruch 1, umfassend einen Koluminator (50), der an einem distalen Ende des ersten Glasfaserbündels angeordnet ist, das so konfiguriert ist, dass es das dadurch in den Körper des Patienten übertragene Licht formt.

5. Vorrichtung nach Anspruch 1, wobei das distale Ende des zweiten Glasfaserbündels so angeordnet ist, dass es das durch biologische und andere Komponenten im Körper des Patienten seitlich gestreute Licht einfängt.

6. Vorrichtung nach Anspruch 5, wobei das distale Ende des zweiten Lichtleitfaserbündels so angeordnet ist, dass es das durch biologische und andere Komponenten im Körper des Patienten rückgestreute, vorwärts gestreute und/oder seitlich gestreute Licht einfängt.

7. Vorrichtung nach Anspruch 1, wobei die Quelle eine Laserdiode (68) oder eine andere Quelle eines kohärenten Lichts ist.

8. Vorrichtung nach Anspruch 7, umfassend eine Schnittstelleneinheit, die entfernt vom Detektor angeordnet und so konfiguriert ist, dass sie von diesem empfangene Informationen anzeigt, und ferner optional:
a) die entfernte Schnittstelle (100b) über eine drahtlose Verbindung mit dem Detektor gekoppelt ist, wobei in diesem Fall die drahtlose Verbindung optional entweder eine Bluetooth- oder eine Infrarotverbindung ist,
b) die entfernte Schnittstelle über eine drahtgebundene Verbindung, eine drahtlose Verbindung oder eine Kombination davon mit dem Detektor gekoppelt ist,
c) die Schnittstelleneinheit so konfiguriert ist, dass sie den Detektor und die Quelle steuert,
d) die entfernte Schnittstelle eine beliebige Größe hat und so konfiguriert ist, dass sie in eine Tasche gesteckt und/oder vom Patienten, einem Gesundheitsdienstleister oder einer anderen Person getragen werden kann.

9. Vorrichtung nach Anspruch 1, die für das in-vivo-Testen von Peritonealflüssigkeit eingerichtet ist, wobei:
a) der Detektor (46) so angeordnet ist, dass er einen Brechungsindex von Flüssigkeit im Peritoneum als Funktion des durch die Flüssigkeit darin gestreuten Lichts bestimmt, wobei
b) der Detektor (46) so angeordnet ist, dass er das von zellgroßen Komponenten verschiedener Typen im Peritoneum gestreute Licht detektiert, so dass diese Komponenten voneinander unterschieden werden können.

## Revendications

1. Appareil (42) destiné au test in vivo de fluides péritonéaux dans le corps d'un patient, comprenant
A. une source d'éclairage (44) et un détecteur d'éclairage (46), tous deux disposés à l'extérieur d'un patient,
B. un premier faisceau de fibres optiques (102) configuré pour transporter l'illuminant provenant de la source d'éclairage dans le péritoine du patient (14) et disposé pour éclairer le fluide péritonéal qui s'y trouve,
C. un second faisceau de fibres optiques (104) (i) ayant une extrémité distale configurée pour capter l'illuminant réfléchi et/ou diffusé (collectivement, « diffusé ») par le fluide péritonéal dans le péritoine du patient et (ii) étant configuré pour transporter cet illuminant diffusé capté jusqu'au détecteur,
D. le détecteur est configuré pour détecter les événements de diffusion dans l'illuminant capté et diffusé transporté par le second faisceau de fibres optiques à une échelle cellulaire de résolution de telle sorte que les globules blancs (WBC) dans le liquide péritonéal peuvent être (i) distingués les uns des autres ainsi que des globules rouges, de la fibrine et d'autres composants du liquide péritonéal, et (ii) comptés, et dans lequel
E. le détecteur comprend un microprocesseur (62) conçu pour analyser les événements de diffusion détectés dans le temps et pour produire une alerte indiquant l'apparition d'une péritonite lorsque l'un quelconque (i) d'un nombre de ces événements varie par rapport à une ligne de base et (ii) d'une tendance de cette variance dans le temps dépasse une quantité sélectionnée.

2. Appareil selon la revendication 1, dans lequel l'un quelconque des premier et second faisceaux de fibres optiques (102 ; 104) est configuré pour être acheminé vers le corps du patient par l'intermédiaire d'un cathéter (19), et,
i) éventuellement, l'un quelconque des premier et second faisceaux de fibres optiques est configuré pour être disposé au moins partiellement dans le cathéter, et, en outre, éventuellement,
ii) le dispositif comprend une pointe (105) configurée pour être disposée à une extrémité distale du cathéter destiné à fixer les extrémités distales des premier et second faisceaux de fibres optiques de telle sorte que ce dernier capte et transmette au détecteur l'illuminant diffusé dans le corps du patient.

3. Appareil selon la revendication 1, comprenant une lentille ou un columinateur (50) disposé(e) à une extrémité proximale du premier faisceau de fibres optiques configuré pour former l'illuminant produit par la source pour le transfert au premier faisceau de fibres optiques.

4. Appareil selon la revendication 1, comprenant un columinateur (50) disposé à une extrémité distale du premier faisceau de fibres optiques configuré pour former l'illuminant transmis alors dans le corps du patient.

5. Appareil selon la revendication 1, dans lequel l'extrémité distale du second faisceau de fibres optiques conçue pour capter l'éclairage diffusé latéralement par des composants biologiques et autres dans le corps du patient.

6. Appareil selon la revendication 5, dans lequel l'extrémité distale du second faisceau de fibres optiques est conçue pour capter l'éclairage d'un composant biologique quelconque ou de tout autre dans le corps du patient, rétrodiffusé, diffusé vers l'avant et/ou diffusé latéralement.

7. Appareil selon la revendication 1, dans lequel la source est une diode laser (68) ou une autre source d'éclairage cohérent.

8. Appareil selon la revendication 7, comprenant une unité d'interface qui est disposée à distance du détecteur et qui est configurée pour afficher les informations reçues de celui-ci, et dans lequel, en outre, éventuellement, l'une quelconque de :
a) l'interface à distance (100b) est couplée au détecteur au moyen d'une liaison sans fil, auquel cas, en outre, éventuellement, la liaison sans fil est l'une quelconque des liaisons Bluetooth et infrarouge,
b) l'interface à distance est couplée au détecteur au moyen d'une liaison filaire, d'une liaison sans fil ou d'une combinaison de celles-ci,
c) l'unité d'interface est configurée pour commander l'un quelconque du détecteur et de la source,
d) l'interface à distance est d'une taille quelconque et configurée pour être glissée sur une poche ou dans celle-ci et/ou portée par le patient, un prestataire de soins de santé ou toute autre personne.

9. Appareil selon la revendication 1, conçu pour le test in vivo de fluide péritonéal, dans lequel l'un quelconque parmi :
a) le détecteur (46) est conçu pour déterminer un indice de réfraction du fluide dans le péritoine en fonction de l'illuminant diffusé par le fluide qui s'y trouve,
b) le détecteur (46) est conçu pour détecter les illuminants dispersés à partir de composants de taille cellulaire de différents types dans le péritoine, de telle sorte que ces composants puissent être distingués les uns des autres.
